# EUROPEAN PATENT APPLICATION

(11) **EP 4 306 035 A1**
(43) Date of publication of application: **17.01.2024**
(21) Application number: 22766705.2
(22) Date of filing: 08.02.2022
(51) Int. Cl.: A61B 1/045, G02B 23/24

(54) **ENDOSCOPE SYSTEM AND METHOD FOR OPERATING SAME**

(30) Priority: 09.03.2021 JP 2021037558
(71) Applicant: FUJIFILM Corporation, Tokyo 106-8620 (JP)
(72) Inventor: IWANE, Kosuke, Ashigarakami-gun, Kanagawa 258-8538 (JP)
(74) Representative: Hughes, Andrea Michelle
(86) International application number: PCT/JP2022/004837
(87) International publication number: WO 2022/190740

(57) **Abstract**

There are provided an endoscope system and a method of operating the same with which a position of a detection target can be specified even in a case where visibility of the detection target is reduced.

In a case where the detection target is detected, the detection target is associated with a landmark (LM) in a landmark setting process, and a detected position display indicator (30) is displayed on a display (18). In a case where the detection target is not detected and the landmark (LM) is detected, a position information estimation process is executed, and an estimated position display indicator (36) is displayed on the display (18).

## Description

### BACKGROUND OF THE INVENTION

### 1. Field of the Invention

The present invention relates to an endoscope system that detects a detection target, such as a bleeding point, and a method of operating the same.

### 2. Description of the Related Art

In a medical field, an endoscope system including a light source device, an endoscope, and a processor device has been widely used. In endoscopic diagnosis, there is a case where a detection target such as a bleeding point is detected during endoscopic treatment. The detection of the detection target has been performed not only by detection by visual observation but also estimation by comparison with a past image. WO2019/202827A, JP2015-529489A, and JP2011-036371A disclose that a bleeding point or region is detected from an image.

### SUMMARY OF THE INVENTION

However, it is difficult to specify a position of the detection target by visual observation or from an image in a case where a factor that reduces visibility of the detection target, such as accumulated blood due to bleeding, occurs over time.

An object of the present invention is to provide an endoscope system and a method of operating the same with which a position of a detection target can be specified even in a case where the visibility of the detection target is reduced.

An endoscope system according to an aspect of the present invention comprises: a processor, in which the processor is configured to: acquire an endoscope image; acquire detection target actual position information of a detection target by performing a first detection process on the endoscope image; acquire position information of a landmark by performing a second detection process on the endoscope image in a case where the detection target actual position information is detected; perform a landmark setting process of associating the detection target actual position information with the position information of the landmark; after the landmark setting process, in a case where the detection target actual position information is not acquired and the landmark setting process has been performed, perform a position information estimation process, and calculate detection target estimated position information; and display the detection target estimated position information on a display.

It is preferable that the processor is configured to display the detection target actual position information, the detection target estimated position information, and the position information of the landmark on the display in different modes.

It is preferable that the processor is configured to: perform notification using either or both of a notification sound and notification on the display; and perform the notification in at least one case of a case where the detection target actual position information is detected during the first detection process or a case where the position information of the landmark is detected during the second detection process.

It is preferable that the processor is configured to: perform notification using either or both of a notification sound and notification on the display; and perform the notification in at least one case of a case where the landmark setting process is completed, a case where the detection target is not detected, a case where the position information estimation process is started, or a case where the detection target estimated position information is calculated during the position information estimation process, as a result of the landmark setting process.

It is preferable that the processor is configured to: perform notification using either or both of a notification sound and notification on the display; and perform the notification in a case where a required number of pieces of the position information of the landmarks are not capable of being acquired during the second detection process and the landmark setting process is not capable of being executed, and in a case where the detection target actual position information disappears before the landmark setting process is completed and the landmark setting process fails.

It is preferable that the processor is configured to display, on the display, acquisition number information of position information by notification including at least a quantity of the detection target estimated position information.

It is preferable that the processor is configured to display, on the display, acquisition number information of the detection target actual position information and the position information of the landmark.

It is preferable that the processor is configured to limit the number of landmarks used in the position information estimation process.

It is preferable that the processor is configured to select the landmark to be displayed on the display among the landmarks, and limit the landmarks to be displayed on the display.

It is preferable that the processor is configured to receive a user operation for designating whether or not the landmark is usable in the position information estimation process.

It is preferable that the endoscope image includes a first endoscope image based on first illumination light and a second endoscope image based on second illumination light having a spectrum different from a spectrum of the first illumination light, and the processor is configured to perform the first detection process and the second detection process from the first endoscope image, and display the detection target actual position information, the detection target estimated position information, and the position information of the landmark on the display from the second endoscope image.

It is preferable that a start timing of the first detection process is any of a time at which water supply is detected, a time at which incision is detected, a time at which a treatment tool is detected, or a time at which a user operation is performed, an end timing of the first detection process is any of a time at which a predetermined time has elapsed with non-detection of the detection target, a time at which a predetermined time has elapsed since disappearance of the detection target, a time at which a bleeding region is in a non-enlargement state, or a time at which a user operation is performed, a start timing of the second detection process is a time at which the detection target is detected, an end timing of the second detection process is any of a time at which the detection target disappears, a time at which a required number of the landmarks are not detected within a predetermined time, or a time at which a user operation is performed, a start timing of the position information estimation process is a time at which the landmark setting process is completed and the detection target disappears, and an end timing of the position information estimation process is any of a time at which the landmark disappears, a time at which a hemostasis point is detected, a time at which bleeding around an estimated position of the detection target is not detected, or a time at which a user operation is performed.

It is preferable that a restart timing of the first detection process is any of a time at which the landmark setting process fails, the position information estimation process, or a time at which a user operation is performed.

It is preferable that the landmark is at least any of a mucous membrane pattern, a shape of an organ, or marking by a user operation.

An endoscope system according to the aspect of the present invention comprises: a processor, in which the processor is configured to: acquire an endoscope image; acquire detection target actual position information of a detection target by performing a first detection process on the endoscope image; acquire position information of a landmark by performing a second detection process on the endoscope image; perform a landmark setting process of setting a relative relationship by associating any of the detection target actual position information or detection target estimated position information obtained from a position information estimation process based on the position information of the landmark, with the position information of the landmark each time the endoscope image is updated and the detection target actual position information or the detection target estimated position information is acquired; and display the detection target actual position information or the detection target estimated position information on a display.

It is preferable that the processor is configured to: in a case where a new landmark is detected by acquiring the endoscope image of a new frame in a state where the position information estimation process is continued, perform a new landmark setting process of setting a new relative relationship by associating the detection target estimated position information with the new landmark as the landmark setting process; after the new landmark setting process, in a case where the landmark necessary for the position information estimation process is not recognized, perform a position information estimation process based on the new relative relationship, and calculate a new detection target estimated position information; and display the new detection target estimated position information on the display.

It is preferable that the new landmark is at least any of a mucous membrane pattern, a shape of an organ, or marking by a user operation.

A method of operating an endoscope system according to the aspect of the present invention comprises: via a processor, a step of acquiring an endoscope image; a step of acquiring detection target actual position information of a detection target by performing a first detection process on the endoscope image; a step of acquiring position information of a landmark by performing a second detection process on the endoscope image in a case where the detection target actual position information is detected; a step of performing a landmark setting process of associating the detection target actual position information with the position information of the landmark; a step of performing a position information estimation process and calculating detection target estimated position information in a case where the detection target actual position information is not acquired and the landmark setting process has been performed after the landmark setting process; and a step of displaying the detection target estimated position information on a display.

It is preferable that the method of operating an endoscope system further comprises: via the processor, a step of displaying the detection target actual position information, the detection target estimated position information, and the position information of the landmark on the display in different modes.

According to the present invention, it is possible to specify the position of the detection target even in a case where the visibility of the detection target is reduced.

### BRIEF DESCRIPTION OF THE DRAWINGS

Fig. 1 is a schematic view of an endoscope system.
Fig. 2 is a graph showing spectra of violet light V, blue light B, green light G, and red light R.
Fig. 3A is an explanatory diagram showing a mono-light emission mode, and Fig. 3B is an explanatory diagram showing a multi-light emission mode.
Fig. 4 is a block diagram showing functions of an extended processor device.
Fig. 5 is an image diagram in which a detection target that cannot be detected due to a decrease in visibility is estimated by a position information estimation process.
Fig. 6 is an image diagram of a first display control process of showing a landmark detected by a second detection process and a landmark position display indicator.
Fig. 7 is an image diagram in which landmarks used in the position information estimation process are limited.
Fig. 8 is an image diagram for describing landmark setting.
Fig. 9 is an image diagram of a second display control process of showing a landmark position display indicator and a link line in a case where a detection target is not detected after the landmark setting is completed.
Fig. 10 is an image diagram in which a third display control process of displaying an estimated position display indicator calculated by the position information estimation process is added to the second display control process.
Fig. 11 is an explanatory diagram corresponding to an indicator showing each position information.
Fig. 12 is an explanatory diagram showing an estimated position display mode that changes according to a reliability degree of estimated position information.
Fig. 13 is an image diagram for describing a process from a first detection process to a second detection process with notification.
Fig. 14 is an image diagram for describing a process from a landmark setting process to a position information estimation process with notification.
Fig. 15A is an image diagram showing an insufficient number of landmarks, and Fig. 15B is an image diagram showing non-detection of a detection target, in a pattern in which the landmark setting process is not completed.
Fig. 16 is an image diagram in which a detection information display field is expanded on a display.
Fig. 17 is an explanatory diagram related to start and end timings in the first detection process, the second detection process, and the position information estimation process.
Fig. 18 is an explanatory diagram showing that a detection target and a landmark are detected from a second endoscope image and respective position information items are displayed from a first endoscope image.
Fig. 19 is an explanatory diagram showing a series of flows in a tracking mode.
Fig. 20 is an explanatory diagram showing an update of detection of a landmark used in the position information estimation process.

### DESCRIPTION OF THE PREFERRED EMBODIMENTS

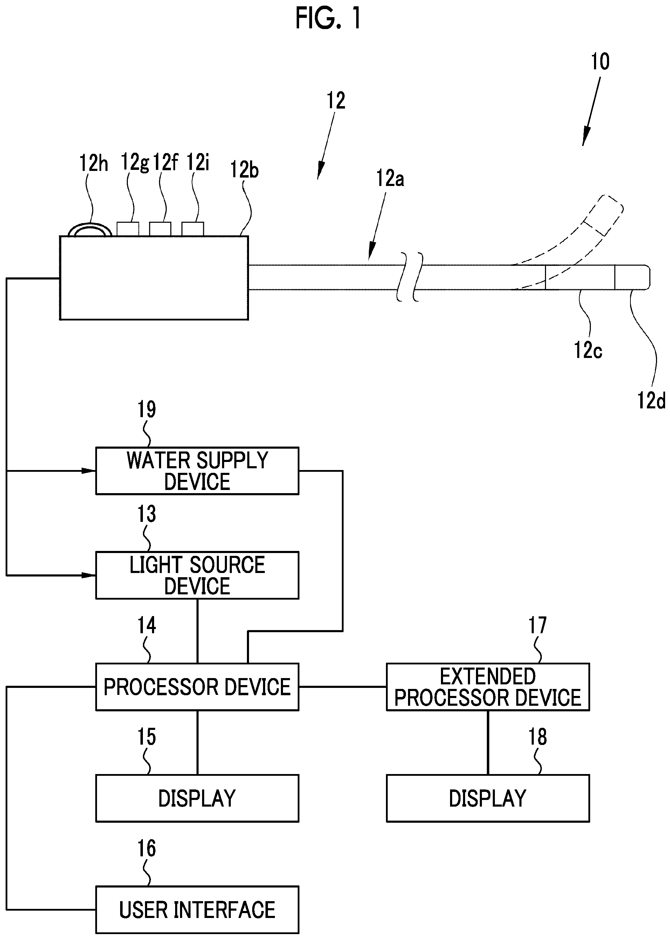

As shown in Fig. 1, an endoscope system 10 includes an endoscope 12, a light source device 13, a processor device 14, a display 15, a user interface 16, an extended processor device 17, a display 18, and a water supply device 19. The endoscope 12 is optically connected to the light source device 13 and is electrically connected to the processor device 14. The light source device 13 supplies illumination light to the endoscope 12. The endoscope 12 is physically connected to the water supply device 19 and is electrically connected to the processor device 14. The water supply device 19 supplies water to the endoscope 12.

The endoscope 12 is used for illuminating an observation target with illumination light and imaging the observation target to acquire an endoscope image. The endoscope 12 includes an insertion part 12a to be inserted into a body of the observation target, an operating part 12b provided at a base end portion of the insertion part 12a, and a bendable part 12c and a distal end part 12d provided on a distal end side of the insertion part 12a. The bendable part 12c performs a bending operation by operating the operating part 12b. The distal end part 12d irradiates the observation target with illumination light and receives reflected light from the observation target to image the observation target. The distal end part 12d is directed in a desired direction by the bending operation of the bendable part 12c. The operating part 12b includes a mode selector switch 12f used for a mode switching operation, a still image acquisition instruction switch 12g used for providing an instruction of acquisition of a still image of the observation target, a zoom operation part 12h used for an operation of a zoom lens 21b, and a water supply switch 12i used for a water supply operation.

The processor device 14 is electrically connected to the display 15 and the user interface 16. The processor device 14 receives the endoscope image from the endoscope 12. The display 15 outputs and displays an image, information, or the like of the observation target processed by the processor device 14. The user interface 16 includes a keyboard, a mouse, a touch pad, a microphone, and the like, and has a function of receiving an input operation such as function setting. The extended processor device 17 is electrically connected to the processor device 14. The extended processor device 17 receives the image or various kinds of information from the processor device 14. The display 18 outputs and displays an image, information, or the like processed by the extended processor device 17.

The endoscope system 10 comprises a mono-light emission mode, a multi-light emission mode, and a tracking mode, and the modes are switched by the mode selector switch 12f. The mono-light emission mode is a mode in which the observation target is continuously illuminated with illumination light having the same spectrum. The multi-light emission mode is a mode in which the observation target is illuminated while switching a plurality of illumination light beams having different spectra according to a specific pattern. The illumination light includes normal light (broadband light such as white light) used for giving brightness to the entire observation target to observe the entire observation target, or special light used for emphasizing a specific region of the observation target. Further, in the mono-light emission mode, the illumination light may be switched to illumination light having a different spectrum by the operation of the mode selector switch 12f. For example, first illumination light and second illumination light having different spectra may be switched. The tracking mode is not exclusive of the mono-light emission mode and the multi-light emission mode, and the tracking can be performed even in the mono-light emission mode and the multi-light emission mode.

The tracking mode is a mode in which actual position information of a detection target is detected, and position information of the detection target and position information of a landmark associated with the position information of the detection target are displayed on the display 18 (or the display 15) in order to allow a user to grasp a position of the detection target such as a bleeding point BS even though a change occurs in an image of a subject or the like.

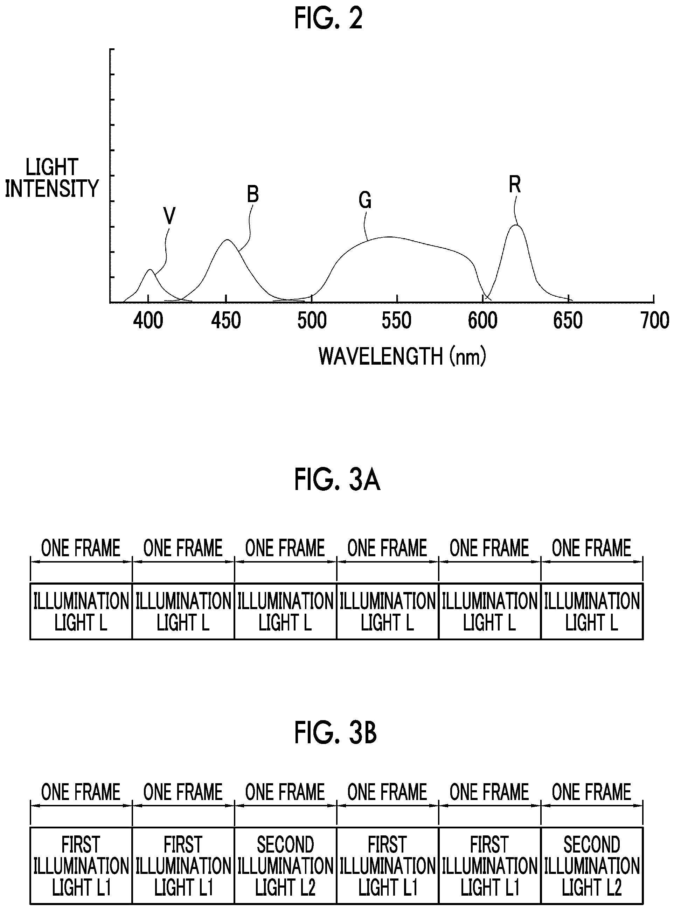

As shown in Fig. 2, the illumination light is preferably emitted by a combination of violet light V, blue light B, green light G, and red light R. The violet light V preferably has a central wavelength of 405±10 nm and a wavelength range of 380 to 420 nm. The blue light B preferably has a central wavelength of 450±10 nm and a wavelength range of 420 to 500 nm. The green light G preferably has a wavelength range of 480 to 600 nm. The red light R preferably has a central wavelength of 620 to 630 nm and a wavelength range of 600 to 650 nm.

The light source device 13 independently controls the light amounts of the four colors of violet light V, blue light B, green light G, and red light R. As shown in Fig. 3A, in the case of the mono-light emission mode, illumination light L having the same spectrum is continuously emitted for each frame. On the other hand, in the case of the multi-light emission mode, control is performed to change the light amounts of the four colors of violet light V, blue light B, green light G, and red light R in accordance with a specific pattern. For example, as shown in Fig. 3B, as the specific pattern, there is a pattern in which a first light emission pattern in which first illumination light L1 having a first spectrum is emitted for two consecutive frames and a second light emission pattern in which second illumination light L2 having a second spectrum different from the first spectrum is emitted for one frame are alternately performed. The frame refers to a time from when an imaging sensor (not shown) provided in the distal end part 12d of the endoscope starts receiving reflected light from the observation target to when output of charge signals accumulated based on the light reception is completed.

The processor device 14 may implement a function of the extended processor device 17 and replace the extended processor device 17. The processor device 14 performs various kinds of processing executed by functions implemented by the extended processor device 17 described below, in addition to a function of receiving an endoscope image from the endoscope 12. In this case, an image or information subjected to various kinds of processing may be displayed on the display 15 or may be displayed on the display 18.

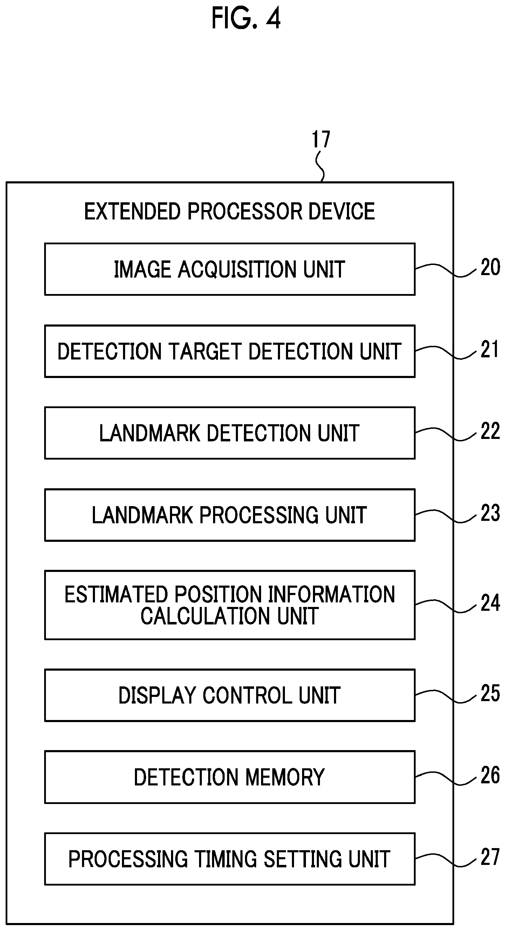

As shown in Fig. 4, the extended processor device 17 comprises an image acquisition unit 20, a detection target detection unit 21, a landmark detection unit 22, a landmark processing unit 23, a display control unit 24, an estimated position information calculation unit 25, a detection memory 26, and a processing timing setting unit 27. The extended processor device 17 is provided with a program memory that stores programs related to various kinds of processing. The program is executed by a processor provided in the extended processor device 17 to implement functions of the image acquisition unit 20, the detection target detection unit 21, the landmark detection unit 22, the landmark processing unit 23, the display control unit 24, the estimated position information calculation unit 25, and the processing timing setting unit 27. The display control unit 24 may perform display control of the display 15 in addition to display control of the display 18.

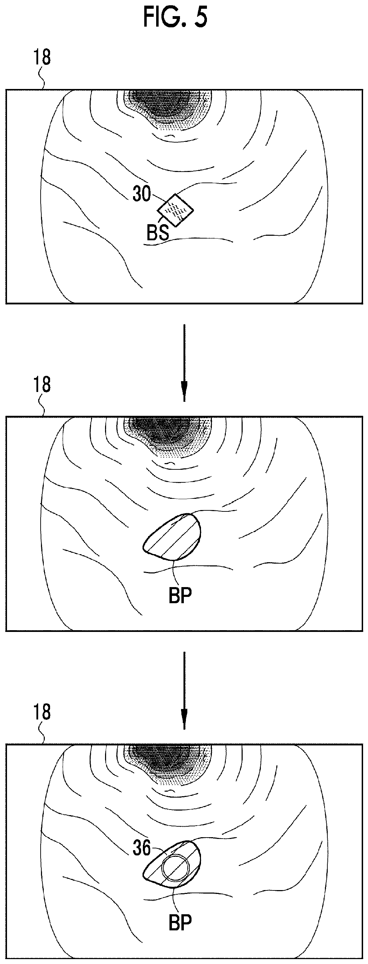

As shown in Fig. 5, even in a case where a detection target such as the bleeding point BS detected in the endoscope image of the display 18 is not detected due to bleeding or the like, the extended processor device 17 can estimate a position of the detection target by performing a position information estimation process of the detection target in a case where a landmark LM is detected. A result of the position information estimation process is shown on the endoscope image by an estimated position display indicator 36 as a detection target estimated position information.

The image acquisition unit 20 acquires the endoscope image transmitted from the processor device 14. The processor device 14 transmits the endoscope image to the extended processor device 17 for each frame. The image acquisition unit 20 acquires the endoscope image for each frame transmitted from the processor device 14.

The detection target detection unit 21 detects a detection target and acquires actual position information of the detection target by performing a first detection process on the endoscope image. As shown in Fig. 5, in a case where the bleeding point BS, which is one of the detection targets, is detected by a first detection process in the endoscope image of the display 18, the display control unit 24 displays a detected position display indicator 30 around the position of the bleeding point BS on the display 18. The position indicated by the detected position display indicator 30 is an actual position of the bleeding point BS. The detection target is preferably at least one of the bleeding point BS, a lesion part such as cancer, a lesion part emphasized by chemical fluorescence (photodynamic diagnosis (PDD)), a shape of a specific organ, or a mucous membrane pattern.

It is preferable that the detection target detection unit 21 is a trained model that has been trained through machine learning using teacher image data including the detection target. The machine learning includes supervised learning, semi-unsupervised learning, unsupervised learning, reinforcement learning, deep reinforcement learning, learning using a neural network, deep learning, and the like. In a case where the detection target is detected by the first detection process, information on the detected detection target is stored in the detection memory 26.

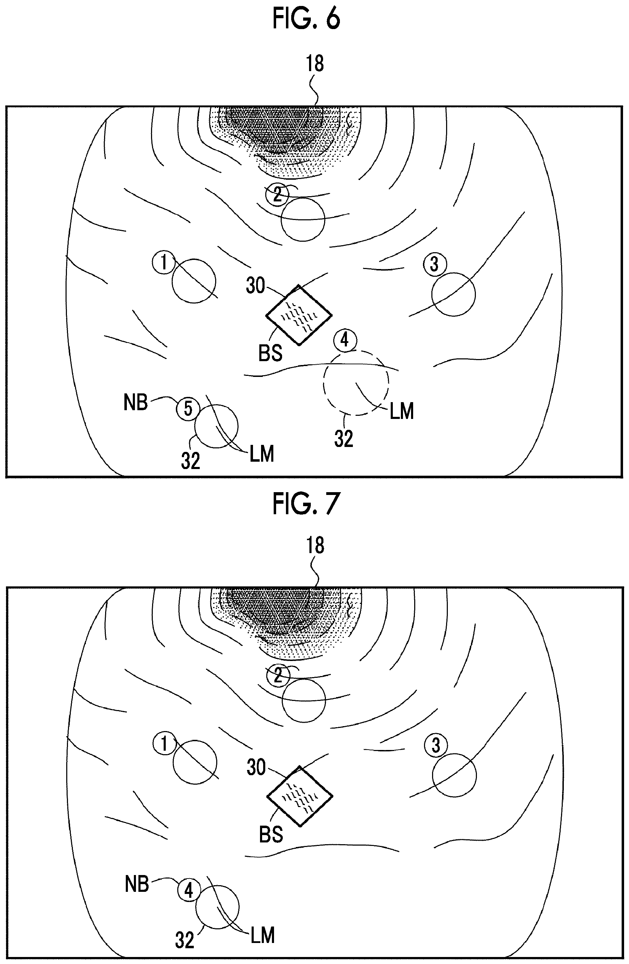

In a case where the detection target is detected by the detection target detection unit 21, the landmark detection unit 22 detects the landmark LM and acquires position information of the landmark LM by performing a second detection process on the endoscope image. Examples of the landmark LM include various structures such as blood vessels and glandular structures, a mucous membrane pattern, a shape of an organ, marking by a user operation, marking after cauterization, and marking given to a body (marking given by a coloring agent or marking with lame or marker given on a bulging material to be injected in a case of incision). As shown in Fig. 6, in a case where the landmark LM is detected in the second detection process in the endoscope image in which the bleeding point BS, which is one of the detection targets, is detected in the first detection process, a landmark position display indicator 32 is displayed on the display 18 at the position of the landmark LM. It is necessary to detect a plurality of landmarks LM in order to execute the position information estimation process, and in this case, it is preferable to be able to distinguish the landmark position display indicators 32 from each other. For example, a number NB (distinction number) for distinction is assigned to each landmark position display indicator 32. The landmark LM is preferably detected not only in the vicinity of the detection target such as a bleeding region BP, but also from a position away from the detection target in order to eliminate a factor that reduces the visibility of the detection target, such as accumulated blood flowing out from the bleeding point BS.

The landmark LM detected in the second detection process is subjected to a landmark setting process of calculating estimated position information of the bleeding point BS using position information of the plurality of landmarks LM by the landmark processing unit 23. However, a use of the landmark LM with low reliability may cause the estimated position of the bleeding point BS to be far away from the actual position. In order to prevent this, the number of the landmarks LM used in the landmark setting process may be limited. For example, as shown in Fig. 7, in a case where an upper limit of the number of the landmarks used in the landmark setting process is set to 4 for the endoscope image in which five landmarks LM are detected, the landmark LM having the distinction number "4" with the lowest reliability degree among the five landmarks LM in Fig. 6 is excluded and is not displayed, the distinction number of the landmark LM detected with the distinction number "5" is displayed as "4", and the landmark setting process is performed from the four landmarks LM. In addition to the upper limit number, a threshold value of the reliability may be set for limitation. In the selection of the landmarks LM to be displayed on the display 18, it is preferable that the landmarks LM are selected so as to surround the detection target instead of being clustered in one portion on a screen, and the number of the landmarks LM is limited by being selected from the landmarks useful for estimation.

Regarding the limitation of the landmarks LM to be detected, the landmark LM with a high reliability degree may be automatically detected by setting the upper limit number or the threshold value, but the landmark LM with low reliability may be designated by a user operation and excluded from the target of the landmark setting process. For example, the landmark LM having the distinction number "4" indicated as having low reliability in a display mode of the landmark position display indicator 32 as shown in Fig. 6 is selected by the user operation through the user interface 16 and is set to be excluded. The position information estimation process is performed with the remaining landmarks LM. The limit number of landmarks LM may be set before the tracking mode is turned ON, or may be set during the tracking mode including the second detection process.

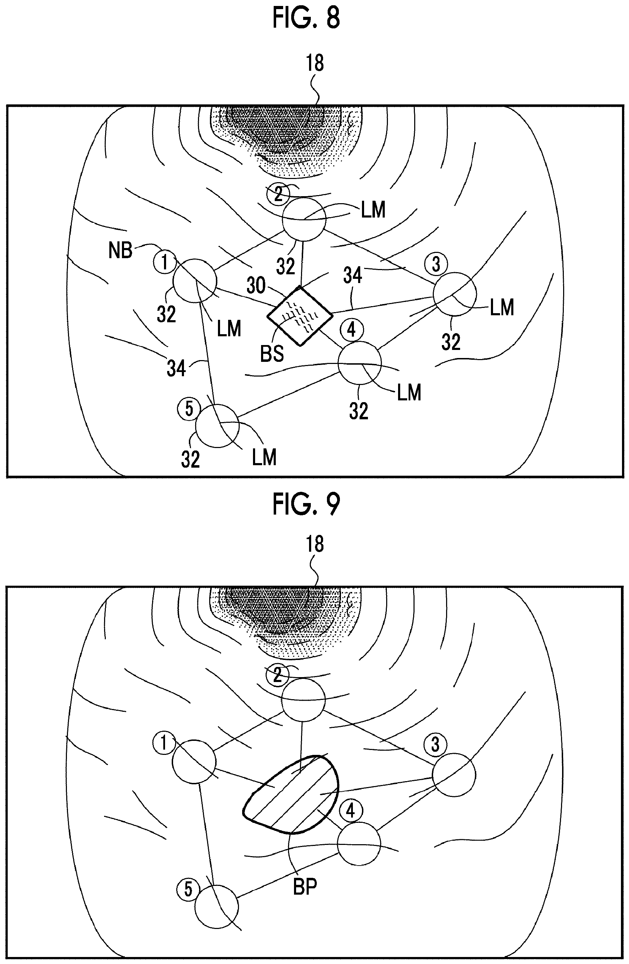

In a case where the required number or more of the landmarks LM are detected by the second detection process, the landmark processing unit 23 performs the landmark setting process of associating the position information of the landmark LM with the actual position information of the detection target. As shown in Fig. 8, in the landmark setting process, as a method of associating the position information of the landmark LM with the actual position information of the detection target, the detected position display indicator 30 and the landmark position display indicator 32 are connected with a link line 34. In this case, it is preferable to associate the position information of the landmark LM detected around the detection target among the landmarks LM with the actual position information of the detection target. That is, in the case of Fig. 8, the landmark position display indicators 32 having the distinction numbers "1", "2", "3", "4", and "5" around the detected position display indicator 30 detected in Fig. 6 need to be connected to at least the detected position display indicator 30 with the link line 34. In addition, it is preferable to connect the landmark position display indicators 32 to each other with the link line 34. Information relating to the position information of the landmark LM and the actual position information of the detection target associated by the landmark setting process is stored in the detection memory 26.

The display control unit 24 performs any of a first display control process of displaying the actual position information of the detection target and the position information of the landmark LM on the display 18 in a case where the detection target is detected, a second display control process of displaying the position information of the landmark LM on the display 18 in a case where the detection target is not detected and the landmark LM is detected, or a third display control process of displaying the estimated position information of the detection target in a case where the detection target is not detected and the landmark setting process is performed. As shown in Fig. 8, by the first display control process, in the endoscope image of the display 18, the detected position display indicator 30 is displayed as the actual position information of the detection target, and the landmark position display indicator 32 is displayed as the position information of the landmark LM. It is preferable that the link line 34 is also displayed on the display 18 in the first display control process.

Then, as shown in Fig. 9, in a case where the display of the detection target disappears from the endoscope image due to a case where a large amount of blood (blood pool) flows out from the bleeding point BS and the bleeding region BP spreads, the detection target is not detected by the detection target detection unit 21. Even in a case where the display of the detection target disappears as described above, the detection of the landmark LM is maintained by the landmark processing unit 23 in a case where the landmark LM remains in the endoscope image. In this case, by performing the second display control process, the landmark position display indicator 32 is displayed on the display 18 as the position information of the landmark LM even though the detected position display indicator 30 is not displayed. It is preferable that the link line 34 is also displayed on the display 18 in the second display control process.

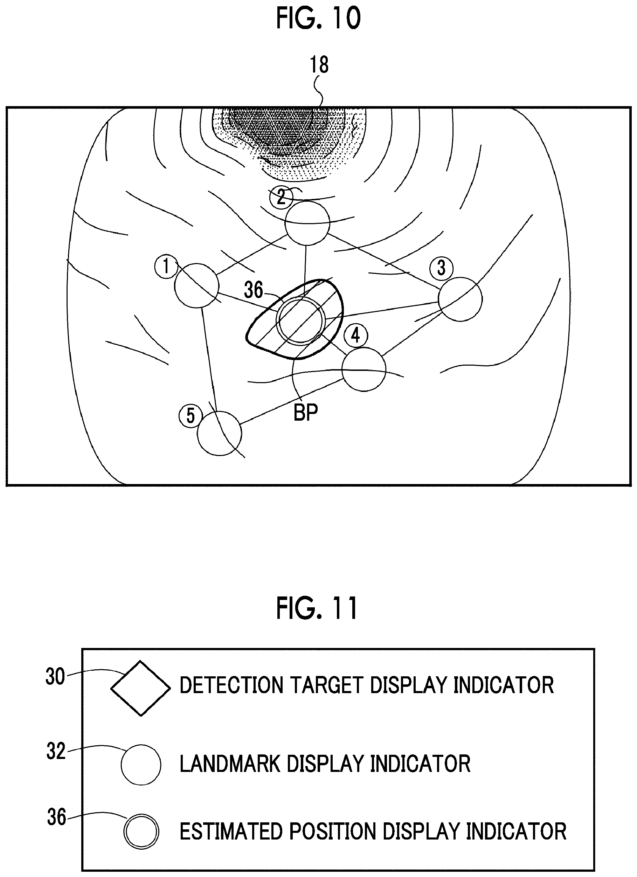

In a case where the detection target is not detected and the landmark setting process has been performed, the estimated position information calculation unit 25 calculates the estimated position information of the detection target by the position information estimation process based on the position information of the landmark LM. As shown in Fig. 9, in a case where the display of the detection target disappears, the display control unit 24 maintains the display of the landmark position display indicator 32 on the display 18 by the second display control process. In this case, as a result of performing the position information estimation process, as shown in Fig. 10, the third display control process of displaying the estimated position display indicator 36 at a position calculated that the detection target exists as the estimated position information of the detection target is added. In the third display control process, the estimated position information of the detection target is displayed as the estimated position display indicator 36, but may be displayed simultaneously with the landmark LM while the second display control process is continued. It is preferable to perform estimation from a positional relationship between the landmark position display indicators 32, for example, a shape of a link formed from the link line 34 in the position information estimation process. In a case where the second display control process is not performed and only the third display control process is performed, as shown in Fig. 5, the landmark position display indicator 32 and the link line 34 are not displayed, and only the estimated position display indicator 36 is displayed.

The detected position display indicator 30, the landmark position display indicator 32, and the estimated position display indicator 36 are displayed on the display 18 in different display modes in order to be easily distinguished from each other. As shown in Fig. 11, a periphery of the detected position display indicator 30 is a rectangle, a periphery of the landmark position display indicator 32 is a circle, and a periphery of the estimated position display indicator 36 is a double circle (see also figures other than Fig. 11). In a case where the detected position display indicator 30 and the estimated position display indicator 36 have the same or very similar display modes, in a case where there is erroneous detection of the detection target, there is a concern that the estimated position display indicator 36 calculated based on information on the erroneous detection cannot be recognized as the erroneous detection.

A processing unit for performing the second process in the landmark processing unit 23 is preferably a trained model for landmark detection that has been trained through machine learning using teacher image data including the landmarks LM. In a case where the landmark processing unit 23 can calculate the reliability degree related to the detection of the landmark LM, it is preferable to change a display mode (color, line style, or the like) of the position information of the landmark LM according to the reliability degree. For example, as shown in Fig. 6, in a case where the reliability degree of the landmark LM having the distinction number "4" is lower than the reliability degrees of the other landmarks LM, it is preferable to make a display mode (dotted line in Fig. 6) of the landmark position display indicator 32 of the landmark LM having the distinction number "4" different from display modes (solid lines in Fig. 8 and the like) of the landmark position display indicators 32 of the other landmarks LM.

In the estimated position display control process, it is preferable to change an estimated position display mode, which is the estimated position display indicator 36 in the present embodiment, according to the reliability degree of the estimated position information. In this case, the estimated position information calculation unit 25 calculates the reliability degree of the estimated position information in accordance with the calculation of the estimated position information. For example, it is preferable to calculate a confidence degree for the estimated position information as a confidence degree for the estimated position information from a model that has been trained through machine learning. The user can select an operation on the observation target according to the reliability degree of the estimated position information. For example, in a case where the reliability degree is high, a hemostasis process for the bleeding point BS is performed, whereas in a case where the reliability degree is low, the hemostasis process is not performed in order to avoid hemostasis at a wrong portion.

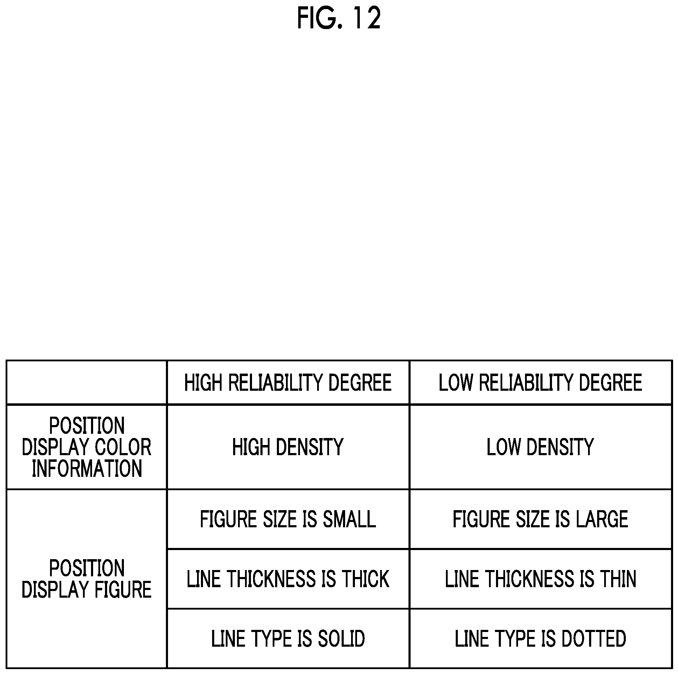

Specifically, as shown in Fig. 12, in a case where the estimated position information is represented by the position display color information, the density of the position display color information is increased in a case where the reliability degree of the estimated position information is high reliability equal to or higher than a predetermined value, and the density of the position display color information is decreased in a case where the reliability degree of the estimated position information is low reliability less than the predetermined value. Further, in a case where the estimated position information is represented by the position display figure, the size of the position display figure is reduced in the case of high reliability degree, and the size of the position display figure is increased in the case of low reliability degree. In the case of high reliability degree, the line thickness of the position display figure is increased, and in the case of low reliability degree, the line thickness of the position display figure is reduced. Further, in the case of high reliability degree, the line type of the position display figure is set to a solid line, and in the case of low reliability degree, the line type of the position display figure is set to a dotted line. As described above, the reliability degree of the estimated position information can be intuitively grasped by changing the estimated position display mode according to the reliability degree.

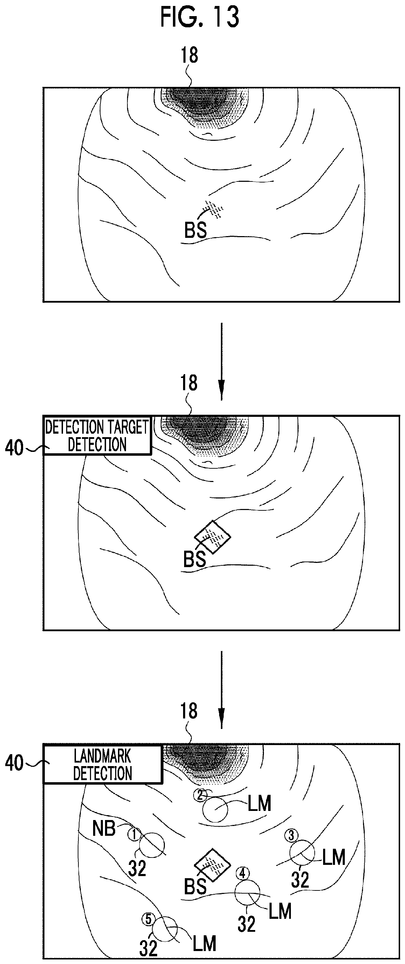

The display control unit 24 may perform notification of a result of each process using a notification sound and notification on the display 18. As shown in Fig. 13, in a case where the detection target is confirmed, for example, as a result of detecting the bleeding point BS as the detection target by the first detection process from the endoscope image in which the bleeding point BS exists, notification of "detection target detection" may be performed, and in a case where the landmark LM is detected by the second detection process, notification of "landmark detection" may be performed by expanding a notification field 40 on the display 18 together with the display of the landmark position display indicator 32. The notification field 40 does not always needs to be expanded, and is temporarily displayed for 5 seconds or the like in a case where a notification reason occurs. In addition, the notification content may be provided not only by the display of the display 18 but also by a notification sound. Hereinafter, an example of notification will be described separately for a case where landmark setting is completed and a case where landmark setting is not completed, which are conditions for starting the position information estimation process to be executed in a case where the detection target is not detected.

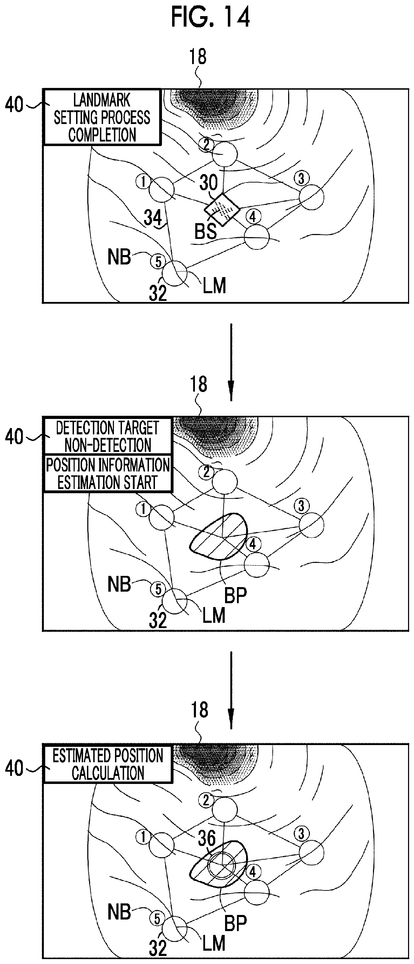

In a case where the landmark setting process is completed, as shown in Fig. 14, the detection target detected in Fig. 13 and a sufficient number of the landmarks LM are associated with each other, and in a case where the link line 34 is formed between the landmark position display indicators 32, notification of "landmark setting process completion" is performed on the display 18. In addition, in a case where the landmark setting process is completed and the detection target is not detected due to the bleeding region BP or the like, notification of "detection target non-detection" and "estimated position calculation start" is performed, and the position information estimation process is performed. In a case where the position information estimation process is performed and the estimated position information is calculated, notification of "estimated position calculation" is performed and the estimated position display indicator 36 is displayed at the estimated position of the detection target.

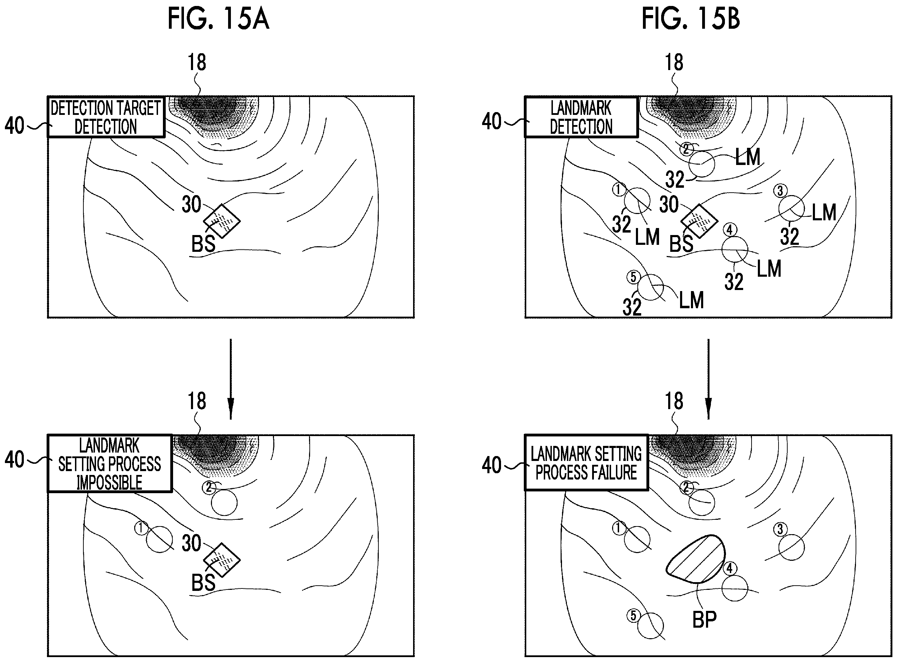

In a case where the landmark setting process is not completed, there are, for example, a pattern (Fig. 15A) in which a predetermined time has elapsed without a required number of the landmarks LM being sufficient for the landmark setting process, in which notification of "landmark setting process impossible" is performed in the notification field 40, and a pattern (Fig. 15B) in which the detection target is not detected due to the blood pool BP or the like in the middle of the landmark setting process, in which notification of "landmark setting process failure" is performed. The required number of the landmarks LM for the landmark setting process is the number that can surround the detection target, and is at least three landmarks LM. Further, the required number may be set to four or more by a user operation or the like. In a case where the landmark setting process cannot be executed, it is preferable to restart from the first detection process or the second detection process.

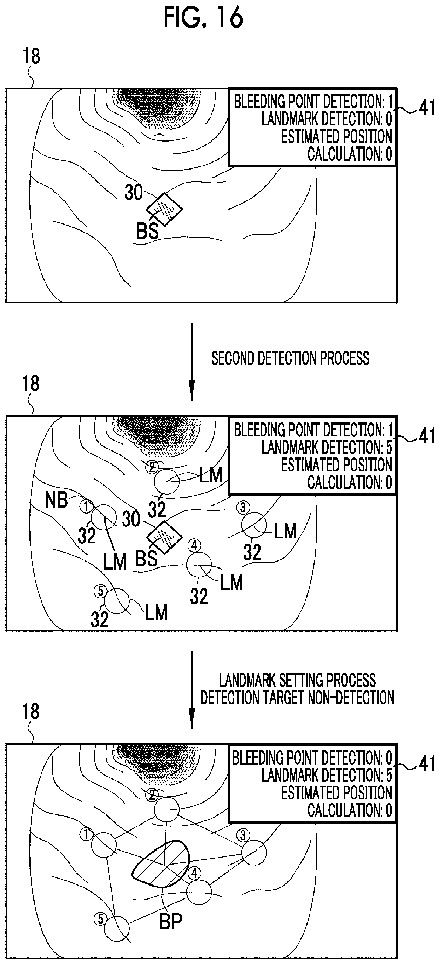

Further, the acquisition number of the position information in the tracking mode may be displayed on the display 18. For example, as shown in Fig. 16, an acquisition number information display field 41 for displaying the acquisition number of the position information in the tracking mode is expanded at the upper right of the display 18. It is preferable that the acquisition number information display field 41 is always expanded to display the number of positions of the detection target or the landmark LM detected by the first detection process or the second detection process during the tracking mode and the acquisition number of the estimated position information of the detection target calculated by the position information calculation process, as characters. For example, in a case where one bleeding point BS is detected as a result of the first detection process, "bleeding point detection number: 1", "landmark detection number: 0", and "estimated position calculation number: 0" are displayed, and in a case where five landmarks LM are detected as a result of the subsequent second detection process, "bleeding point detection number: 1", "landmark detection number: 5", and "estimated position calculation number: 0" are displayed. In a case where the landmark setting is completed but the detection target is not detected, "bleeding point detection number: 0", "landmark detection number: 5", and "estimated position calculation number: 0" are displayed. The display of the acquisition number is updated every time the acquisition number of the position information varies. Further, with respect to the process being executed other than the acquisition number, the content of the process in progress such as "first detection process in progress", "landmark setting process in progress", and "position information estimation process in progress" may be displayed. Note that only one of the notification field 40 and the acquisition number information display field 41 in Fig. 13 to Fig. 15B may be used, or both may be used in combination. The acquisition number may include the quantity of the detection target and the quantity of the landmark in addition to the quantity of the estimated position information.

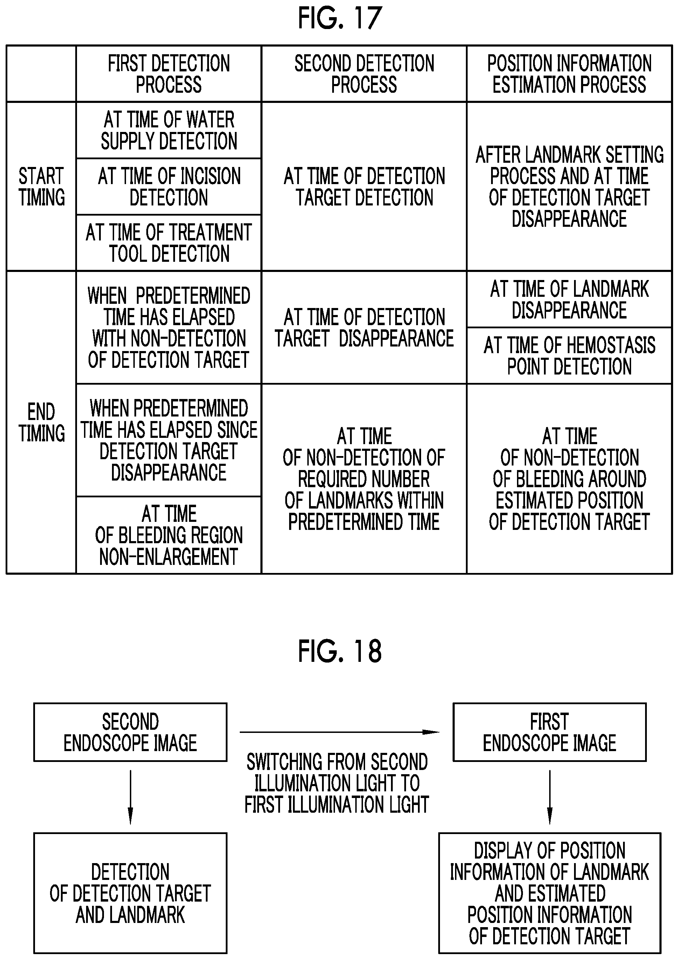

It is preferable that the processing timing setting unit 27 sets a start timing or an end timing of the first detection process, a start timing or an end timing of the second detection process, and a start timing or an end timing of the position information estimation process (see Fig. 17). By setting the start timing and the end timing in this way so that the first detection process or the position information estimation process is not always performed, it is possible to suppress the erroneous detection. In addition, in a case where it is determined that the first detection process or the position information estimation process is not necessary, the actual position information and the estimated position information of the detection target are not displayed, and thus it is possible to make it easy to see a part other than the detection target such as the bleeding point BS.

Specifically, as shown in Fig. 17, it is preferable that the start timing of the first detection process is a timing at which water supply sent from the distal end part of the endoscope 12 to the observation target is detected in the endoscope image (at the time of water supply detection), a timing at which incision made in a part of the observation target by a treatment tool or the like is detected in the endoscope image (at the time of incision detection), or a timing at which the treatment tool protruding from the distal end part of the endoscope 12 is detected in the endoscope image (at the time of treatment tool detection). In addition, it is preferable that the end timing of the first detection process is a timing at which a predetermined time has elapsed without detection of the detection target or the bleeding (at the time of failure of the first detection process), a timing after a predetermined time has elapsed from a timing at which the detected detection target disappears (after an elapse of a predetermined time from disappearance of the detection target), a timing at which a hemostasis point is detected (at the time of hemostasis point detection), or a timing at which a predetermined time has elapsed without increasing the bleeding region BP (at the time of bleeding region non-enlargement). In a case where the first detection process ends, the observation target may be changed. A bleeding region supplement unit (not shown) having a function of capturing a change in a region such as the position or enlargement of the bleeding region BP may be provided.

It is preferable that the start timing of the second detection process is a timing at which the detection target is detected by the first detection process (at the time of detection target detection). In addition, it is preferable that the end timing of the second detection process is a timing after a predetermined time has elapsed from the detection of the latest landmark LM (after an elapse of a predetermined time after the detection of the landmark) or a timing at which the number of the detected landmarks LM reaches a set upper limit number (after the number of the detected landmarks reaches the upper limit number).

The start timing of the position information estimation process is a timing at which the landmark is set and the detection target disappears. It is preferable that the end timing of the position information estimation process is a timing at which the estimated position information cannot be calculated by the position information estimation process due to disappearance of the landmark (at the time of failure of the position information estimation process), a timing at which a hemostasis point is detected as a result of performing a hemostatic treatment on the bleeding point with a treatment tool or the like (at the time of hemostasis point detection), or a timing at which bleeding cannot be confirmed in the estimated position display indicator 36 and its periphery (at the time of bleeding non-detection at the estimated position). In a case where the tracking mode is continued even though the position information estimation process fails, it is preferable to redo the first detection process.

It is preferable that the re-detection start timing of the first detection process is a timing at which the detection target disappears before the landmark setting process is completed (at the time of failure of the landmark setting process) or a timing at which the landmark disappears before the position information estimation process is completed (at the time of failure of the position information estimation process).

In the tracking mode, as shown in Fig. 18, it is preferable that the detection target and the landmark LM are detected from the second endoscope image based on the second illumination light to perform the landmark setting process, and then the second illumination light is switched to the first illumination light, and the estimated position information of the detection target and the position information of the landmark LM are displayed on the first endoscope image based on the first illumination light. Accordingly, the position or the region of the detection target is not missed in a case where the second illumination light is switched to the first illumination light. It is preferable that the second illumination light is light suitable for detecting the detection target, for example, special light including violet light capable of highlighting a structure. On the other hand, it is preferable that the first illumination light is light suitable for displaying the actual position information of the detection target, the position information of the landmark LM, and the estimated position information of the detection target, for example, white light.

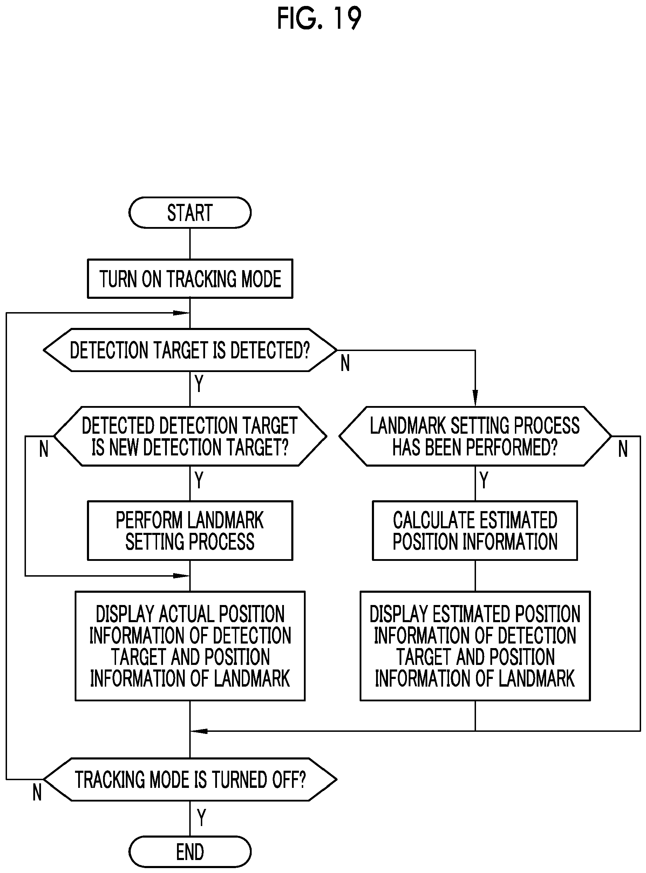

Next, a series of flows in the tracking mode will be described with reference to a flowchart of Fig. 19. The tracking mode is turned ON by operating the mode selector switch 12f. Accordingly, the first detection process is performed on the endoscope image. In a case where the detection target including the bleeding point BS is detected in the first detection process, the actual position information of the detection target is acquired. In a case where the detected detection target is a new detection target, the landmark setting process is performed. In the landmark setting process, the landmark LM is detected and the position information of the landmark LM is acquired by performing the second detection process on the endoscope image. In addition, the position information of the landmark LM is associated with the actual position information of the detection target. The position information of the landmark LM and the actual position information of the detection target which are associated with each other are displayed on the display 18. On the other hand, in a case where the detected detection target is not a new detection target, the position information of the landmark LM and the actual position information of the detection target that have already been associated in the landmark setting process are displayed on the display 18.

Whether or not the detection target is a new detection target is determined depending on whether or not information relating to the detection target is present in the detection memory 26. In a case where a new detection target is detected, the information relating to the detection target that has already been stored in the detection memory 26 is deleted, and information relating to the new detection target is newly stored in the detection memory 26. In this case, it is preferable that information relating to the landmark LM associated with the detection target to be deleted is also deleted.

On the other hand, in a case where the detection target cannot be detected by the first detection process, it is determined whether or not the landmark setting process has already been performed (determination of whether or not the landmark setting process is being performed). In a case where the landmark setting process has already been performed, the estimated position information of the detection target is calculated based on the position information of the landmark LM. Then, the calculated estimated position information of the detection target and the position information of the landmark LM are displayed on the display 18. The series of processes described above is repeatedly performed as long as bleeding is detected or estimated. Then, in a case where the mode selector switch 12f is operated and the tracking mode is turned OFF, the detection of the detection target and the like are ended.

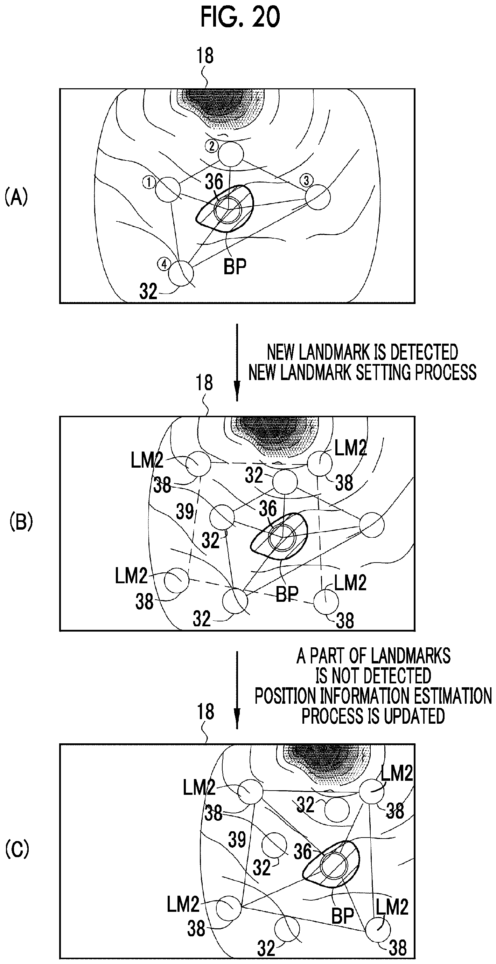

Since the endoscope 12 is manually operated, even though the estimated position of the detection target is continuously captured in the endoscope image, a range of the endoscope image may change, and in a case where the landmark LM surrounding the estimated position of the detection target does not fall within the endoscope image, the organ may be deformed and the relationship between the landmark and the detection target may be changed. As shown in Fig. 20, new landmarks LM2 may be detected at positions different from the positions of the landmarks LM used for the position information estimation process in a case where the endoscope 12 images the next frame, the landmarks LM used for the position information estimation process may be updated, and the display of the estimated position display indicator 36 on the bleeding region BP may be continued.

In a case where the landmark LM used in the position information estimation process is updated, the landmark LM before update is set as the landmark LM and the landmark LM after update is set as the new landmark LM2. (A) of Fig. 20 a displays the estimated position display indicator 36 in the position information estimation process by the landmark LM. In a case where a new frame is acquired, as shown in (B) of Fig. 20, a new landmark LM2 surrounding the estimated position of the detection target is detected in accordance with a moving direction of the preceding and following frame imaging, and a new landmark position display indicator 38 is displayed. Further, a new landmark setting process of associating the new landmark LM2 with the detection target estimated position information is performed to calculate a new relative relationship. The new relative relationship is displayed by a new link line 39. It is preferable that a dotted line or the like is used as the new link line 39, which is less conspicuous than the link line 34 and is not confusing. A number NB (distinction number) for distinguishing the respective landmark position display indicators 32 can also be assigned to the new landmark position display indicator 38, but may not be assigned in a case where the visibility deteriorates. In this case, the number assigned to the landmark position display indicator 32 may also be non-displayed. The new relative relationship may be calculated by using not only the detection target and the new landmark LM2 but also the landmark LM before the update. It is preferable that the landmark LM to be used in a duplicated manner is a position that continues to fall within the endoscope screen with respect to a moving direction of the frame imaging.

After the new landmark setting process, in a case where the endoscope 12 acquires an endoscope image of a new frame and the landmark LM necessary for the position information estimation process is not recognized, as shown in (C) of Fig. 20, a position information estimation process based on the new relative relationship is performed, the detection target estimated position information is calculated, and the estimated position display indicator 36 is displayed on the display 18. Since the position information estimation process by the landmark LM is ended, the link line 34 is not displayed, and the new link line 39 is displayed by a solid line such as the link line 34. In a case where there is a landmark LM that continues to be recognized even after the position information estimation process using the landmark LM ends, the landmark LM may be incorporated into the new relative relationship.

Since the range imaged by the endoscope 12 moves even from the state of the position information estimation process using the new landmark LM2, the update of the landmark LM used for the position information estimation process continues. With the new landmark LM2 as the landmark LM and the new link line 39 as the link line 34, the new landmark LM2 updates the relative relationship by the landmark setting process, and the landmark LM performs the position information estimation process.

In the above-described embodiment, hardware structures of processing units executing various processes, such as the image acquisition unit 20, the detection target detection unit 21, the landmark processing unit 23, the display control unit 24, the estimated position information calculation unit 25, the detection memory 26, or the processing timing setting unit are various processors as follows. The various processors include a central processing unit (CPU) that is a general-purpose processor that executes software (programs) to function as various processing units, a graphical processing unit (GPU), a programmable logic device (PLD) that is a processor capable of changing a circuit configuration after manufacture, such as a field programmable gate array (FPGA), and an exclusive electric circuit that is a processor having a circuit configuration exclusively designed to execute various kinds of processing.

One processing unit may be configured of one of these various processors, or may be configured of a combination of two or more processors of the same type or different types (for example, a plurality of FPGAs, a combination of a CPU and an FPGA, or a combination of a CPU and a GPU). In addition, a plurality of processing units may be constituted by one processor. As an example in which the plurality of processing units are configured of one processor, first, as typified by computers such as a client or a server, one processor is configured of a combination of one or more CPUs and software, and this processor functions as the plurality of processing units. Second, as typified by a system on chip (SoC) or the like, a processor that implements the functions of the entire system including the plurality of processing units by using one integrated circuit (IC) chip is used. As described above, the various processing units are configured using one or more of the various processors as a hardware structure.

Further, the hardware structure of these various processors is more specifically an electric circuit (circuitry) in a form in which circuit elements such as semiconductor elements are combined. The hardware structure of the storage unit is a storage device such as a hard disc drive (HDD) or a solid state drive (SSD).

### Explanation of References

10: endoscope system
12: endoscope
12a: insertion part
12b: operating part
12c: bendable part
12d: distal end part
12f: mode selector switch
12g: still image acquisition instruction switch
12h: zoom operation part
12i: water supply switch
13: light source device
14: processor device
15: display
16: user interface
17: extended processor device
18: display
19: water supply device
20: image acquisition unit
21: detection target detection unit
22: landmark detection unit
23: landmark processing unit
24: display control unit
25: estimated position calculation unit
26: detection memory
27: processing timing setting unit
30: detected position display indicator
32: landmark position display indicator
34: link line
36: estimated position display indicator
38: new landmark display indicator
39: new link line
40: notification field
41: acquisition number information display field
BS: bleeding point
BP: bleeding region
LM: landmark
LM2: new landmark

## Claims

1. An endoscope system comprising:
a processor,
wherein the processor is configured to:
acquire an endoscope image;
acquire detection target actual position information of a detection target by performing a first detection process on the endoscope image;
acquire position information of a landmark by performing a second detection process on the endoscope image in a case where the detection target actual position information is detected;
perform a landmark setting process of associating the detection target actual position information with the position information of the landmark;
after the landmark setting process, in a case where the detection target actual position information is not acquired and the landmark setting process has been performed, perform a position information estimation process, and calculate detection target estimated position information; and
display the detection target estimated position information on a display.

2. The endoscope system according to claim 1,
wherein the processor is configured to display the detection target actual position information, the detection target estimated position information, and the position information of the landmark on the display in different modes.

3. The endoscope system according to claim 1 or 2,
wherein the processor is configured to:
perform notification using either or both of a notification sound and notification on the display; and
perform the notification in at least one case of a case where the detection target actual position information is detected during the first detection process or a case where the position information of the landmark is detected during the second detection process.

4. The endoscope system according to claim 1 or 2,
wherein the processor is configured to:
perform notification using either or both of a notification sound and notification on the display; and
perform the notification in at least one case of a case where the landmark setting process is completed, a case where the detection target is not detected, a case where the position information estimation process is started, or a case where the detection target estimated position information is calculated during the position information estimation process, as a result of the landmark setting process.

5. The endoscope system according to claim 1 or 2,
wherein the processor is configured to:
perform notification using either or both of a notification sound and notification on the display; and
perform the notification in a case where a required number of pieces of the position information of the landmarks are not capable of being acquired during the second detection process and the landmark setting process is not capable of being executed, and in a case where the detection target actual position information disappears before the landmark setting process is completed and the landmark setting process fails.

6. The endoscope system according to any one of claims 1 to 5,
wherein the processor is configured to display, on the display, acquisition number information of position information by character display including at least a quantity of the detection target estimated position information.

7. The endoscope system according to claim 6,
wherein the processor is configured to display, on the display, acquisition number information of the detection target actual position information and the position information of the landmark.

8. The endoscope system according to any one of claims 1 to 7,
wherein the processor is configured to limit the number of landmarks used in the position information estimation process.

9. The endoscope system according to any one of claims 1 to 7,
wherein the processor is configured to select the landmark to be displayed on the display among the landmarks, and limit the landmarks to be displayed on the display.

10. The endoscope system according to any one of claims 1 to 9,
wherein the processor is configured to receive a user operation for designating whether or not the landmark is usable in the position information estimation process.

11. The endoscope system according to any one of claims 1 to 10,
wherein the endoscope image includes a first endoscope image based on first illumination light and a second endoscope image based on second illumination light having a spectrum different from a spectrum of the first illumination light, and
the processor is configured to perform the first detection process and the second detection process from the first endoscope image, and display the detection target actual position information, the detection target estimated position information, and the position information of the landmark on the display from the second endoscope image.

12. The endoscope system according to any one of claims 1 to 11,
wherein a start timing of the first detection process is any of a time at which water supply is detected, a time at which incision is detected, a time at which a treatment tool is detected, or a time at which a user operation is performed,
an end timing of the first detection process is any of a time at which a predetermined time has elapsed with non-detection of the detection target, a time at which a predetermined time has elapsed since disappearance of the detection target, a time at which a bleeding region is in a non-enlargement state, or a time at which a user operation is performed,
a start timing of the second detection process is a time at which the detection target is detected,
an end timing of the second detection process is any of a time at which the detection target disappears, a time at which a required number of the landmarks are not detected within a predetermined time, or a time at which a user operation is performed,
a start timing of the position information estimation process is a time at which the landmark setting process is completed and the detection target disappears, and
an end timing of the position information estimation process is any of a time at which the landmark is not detected, a time at which a hemostasis point is detected, a time at which bleeding around an estimated position of the detection target is not detected, or a time at which a user operation is performed.

13. The endoscope system according to claim 12,
wherein a restart timing of the first detection process is any of a time at which the landmark setting process fails, a time at which the position information estimation process fails, or a time at which a user operation is performed.

14. The endoscope system according to any one of claims 1 to 13,
wherein the landmark is position information of at least any of a mucous membrane pattern, a shape of an organ, or marking by a user operation.

15. An endoscope system comprising:
a processor,
wherein the processor is configured to:
acquire an endoscope image;
acquire detection target actual position information of a detection target by performing a first detection process on the endoscope image;
acquire position information of a landmark by performing a second detection process on the endoscope image;
perform a landmark setting process of setting a relative relationship by associating any of the detection target actual position information or detection target estimated position information obtained from a position information estimation process based on the position information of the landmark, with the position information of the landmark each time the endoscope image is updated and the detection target actual position information or the detection target estimated position information is acquired; and
display the detection target actual position information or the detection target estimated position information on a display.

16. The endoscope system according to claim 15,
wherein the processor is configured to:
in a case where a new landmark is detected by acquiring the endoscope image of a new frame in a state where the position information estimation process is continued, perform a new landmark setting process of setting a new relative relationship by associating the detection target estimated position information with the new landmark as the landmark setting process;
after the new landmark setting process, in a case where the landmark necessary for the position information estimation process is not recognized, perform a position information estimation process based on the new relative relationship, and calculate a new detection target estimated position information; and
display the new detection target estimated position information on the display.

17. The endoscope system according to claim 16,
wherein the landmark and the new landmark are position information of at least any of a mucous membrane pattern, a shape of an organ, or marking by a user operation.

18. A method of operating an endoscope system including a processor, the method comprising:
a step of acquiring an endoscope image;
a step of acquiring detection target actual position information of a detection target by performing a first detection process on the endoscope image;
a step of acquiring position information of a landmark by performing a second detection process on the endoscope image in a case where the detection target actual position information is detected;
a step of performing a landmark setting process of associating the detection target actual position information with the position information of the landmark;
a step of performing a position information estimation process and calculating detection target estimated position information in a case where the detection target actual position information is not acquired and the landmark setting process has been performed after the landmark setting process; and
a step of displaying the detection target estimated position information on a display.

19. The method of operating an endoscope system according to claim 18, further comprising:
via the processor,
a step of displaying the detection target actual position information, the detection target estimated position information, and the position information of the landmark on the display in different modes.
